# EUROPÄISCHE PATENTSCHRIFT

(11) **EP 2 338 536 B1**
(45) Veröffentlichungstag und Bekanntmachung des Hinweises auf die Patenterteilung: **05.08.2015**
(21) Anmeldenummer: 10192869.5
(22) Anmeldetag: 29.11.2010
(51) Int. Cl.: A61L 31/02, A61L 31/08, A61L 31/10, A61L 31/14

(54) **Biokorrodierbare Implantate mit funktionalisierter Beschichtung**
Biocorrodible implants having a functionalized coating
Implants biocorrodables dotés d'un revêtement fonctionnalisé

(30) Priorität: 21.12.2009 US 288348 P
(43) Veröffentlichungstag der Anmeldung: 29.06.2011
(73) Patentinhaber: Biotronik VI Patent AG, 6341 Baar (CH)
(72) Erfinder: Sager, Laura, 8038, Zürich (CH); Adden, Nina, 90427, Nürnberg (DE)
(74) Vertreter: Randoll, Sören

(56) Entgegenhaltungen:
- WO-A1-96/10454
- WO-A1-2008/074227
- WO-A1-2008/077248
- WO-A2-2008/092435
- DE-A1- 10 361 941
- V. GORTEAU ET AL.: "Rigid-rod anion-pi slides for multiion hopping across lipid bilayers", ORG. BIOMOL. CHEM., Bd. 5, 2007, Seiten 3000-3012, XP002716824,

## Beschreibung

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Häufig ist nur eine temporäre Stütz- beziehungsweise Haltefunktion bis zum Abschluss des Heilungsprozesses oder Stabilisierung des Gewebes notwendig beziehungsweise erwünscht. Um Komplikationen zu vermeiden, die aus dem dauerhaften Verbleib der Implantate im Körper resultieren, müssen die Implantate entweder wieder operativ entfernt werden oder sie bestehen aus einem Werkstoff, der allmählich im Körper abgebaut wird, das heißt biokorrodierbar ist. Die Zahl biokorrodierbarer Werkstoffe auf der Basis von Polymeren oder Legierungen ist stetig gewachsen. So sind u. a. biokorrodierbare Metalllegierungen der Elemente Magnesium, Eisen und Wolfram bekannt. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch bildgebende Verfahren, wie z.B. durch Röntgenuntersuchungen erfolgen.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Gebrauch mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe für Stents umfassen Polymere, metallische Werkstoffe und keramische Materialien (zum Beispiel als Beschichtung). Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Insbesondere bei Verwendung von biokorrodierbaren Werkstoffen für Implantate, z.B. Stents, ist die geeignete Kontrolle der Korrosionsgeschwindigkeit des jeweiligen Implantats nach der Implantation im Körper ein Bereich mit weiterem Optimierungsbedarf. Dabei ist die Korrosionsgeschwindigkeit so zu gestalten, dass das Implantat die bestimmungsgemäße Aufgabe oder Funktion im Körper auch über die gewünschte Dauer erfüllen kann. Im Falle eines Stents, sollte die Integrität des Stents für einen Zeitraum gewährleistet sein, der für die Erfüllung des jeweiligen medizinischen Zwecks ausreicht. Andererseits besteht der Vorteil von biokorrodierbaren Stents gerade darin, dass diese Stents nicht unbegrenzt im Körper verbleiben oder sogar wieder operativ entfernt werden müssen, sondern nach einer Weile vom Körper abgebaut und entsorgt werden. Die Integrität eines biokorrodierbaren Stents sollte also über einen Zeitraum gewährleistet sein, der so lange wie nötig und dabei aber so kurz wie möglich ist.

Insbesondere neigen Stents mit einem Grundkörper, der Ganz oder in Teilen aus Eisen oder einer Eisenlegierung besteht, dazu, zu langsam zu korrodieren. Während Stents mit einem Grundkörper, der Ganz oder in Teilen aus Magnesium oder einer Magnesiumlegierung besteht, häufig eine zu schnelle Korrosionsgeschwindigkeit im Körper zeigen.

Es ist bekannt, dass sich die Korrosionsgeschwindigkeit grundsätzlich durch Beschichtung des Implantats beeinflussen lässt. Die für diesen Zweck bislang vorgeschlagenen Beschichtungen reichen allerdings nicht aus und es besteht weiterhin ein Bedarf an neuen Möglichkeiten die Korrosionsgeschwindigkeit von Implantaten, insbesondere Stents, zu beeinflussen.

WO 2008/092435 beschreibt eine biologisch abbaubare Gefäßstütze, welche aus einem inneren bio-degradierbaren metallischen Gerüst und einer äußeren polymeren Beschichtung besteht.

WO 96/10454 beschreibt Lipiddoppelschichten, welche Poly-3-Hydroxybutyrat / Polyphosphat Ionenkanäle enthalten.

WO 2008/074227 offenbart sich selbst bildende Zusammensetzungen, die Ionenkanäle in Lipiddoppelschichten oder Zellmembranen bilden.

In Groteau V. et al (Org. Biomol. Chem., Bd. 5, 3000ff (2007)) werden formstabile Oligo-p-Phenylen-N,N-Naphtalendiimid Kanäle als Anion-π-Rutschen für Chlorid selektive Multi-Ion Sprünge durch Lipiddoppelschichten beschrieben.

Aufgabe der vorliegenden Erfindung ist es mindestens einen der Nachteile des Standes der Technik zu vermindern oder zu vermeiden.

Diese Aufgabe wird gelöst durch Bereitstellung eines Implantates, Implantat, welches ganz oder in Teilen aus einem biokorrodierbaren metallischen Werkstoff besteht und eine Beschichtung aufweist, dadurch gekennzeichnet, dass die Beschichtung mindestens eine Schicht enthält, in die Ionenkanäle eingebettet sind. Die Ionenkanäle liegen in einer Lipiddoppelschicht eingebettet vor.

Bevorzugt ist das erfindungsgemäße Implantat ein Stent.

Der vorliegenden Erfindung liegt die Erkenntnis zugrunde, dass sich die Korrosionsgeschwindigkeit eines Implantates nach der Implantation dadurch beeinflussen und kontrollieren lässt, dass die Konzentration von Ionen am Ort der Korrosion beeinflusst und kontrolliert wird. Dies lässt sich erfindungsgemäß dadurch erreichen, dass das Implantat eine Beschichtung aufweist, die Ionenkanäle umfasst. Durch die Wahl geeigneter Ionenkanäle kann die Konzentration bestimmter Ionen am Ort der Korrosion je nach Bedarf beeinflusst werden. Durch die Wahl geeigneter Ionenkanäle kann die Konzentration ausgewählter Ionen am Ort der Korrosion erhöht werden und dadurch die Korrosionsgeschwindigkeit beeinflusst werden. So weisen beispielsweise biokorrodierbare Implantate enthaltend oder bestehend aus einer Magnesiumlegierung meist Korrosionsgeschwindigkeiten nach der Implantation auf, die zu einem vorzeitigen Verlust der Implantatintegrität führen können. Versieht man solche Implantate mit einer Beschichtung enthaltend Ionenkanäle, die spezifisch durchlässig sind für Ca-Kationen, so lässt sich die Korrosionsgeschwindigkeit des Implantats verlangsamen. Andererseits zeigen Implantate enthaltend oder bestehend aus Reineisen oder einer Eisenlegierung häufig Korrosionsgeschwindigkeiten, die zu einem Abbau des Implantates führen, der zu langsam erfolgt. Werden solche Implantate beispielsweise mit einer Beschichtung versehen enthaltend Ionenkanäle die spezifisch sind für Cl-Anionen, so lässt sich dadurch die Korrosionsgeschwindigkeit solcher Implantate erhöhen.

Das erfindungsgemäße Implantat besteht ganz oder in Teilen aus einem biokorrodierbaren metallischen Werkstoff. Dieser biokorrodierbare Werkstoff ist bevorzugt eine Magnesiumlegierung. Dieser biokorrodierbare Werkstoff kann auch Eisen oder eine Eisenlegierung sein. Vorzugsweise weist das biokorrodierbare Implantat einen metallischen Grundkörper auf. Insbesondere kann der metallische Grundkörper aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung bestehen.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/1, CaCl₂ 0,2 g/1, KCl 0,4 g/l, MgS0₄ 0,1 g/1, NaHCO₃ 2,2 g/1, Na₂HPO₄ 0,126 g/1, NaH₂PO₄ 0,026 g/1). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Erfindungsgemäß weist das Implantat eine Beschichtung auf. Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 10 µm, besonders bevorzugt 2 nm bis 50 nm. Die Beschichtung kann direkt auf die Implantatoberfläche aufgetragen werden. Die Verarbeitung kann nach Standardverfahren für die Beschichtung erfolgen. Bevorzugt wird die Beschichtung durch Tauchen aufgetragen. Es können einschichtige, aber auch mehrschichtige Systeme (zum Beispiel so genannte base coat -, drug coat - oder top coat - Schichten) erstellt werden. Die Beschichtung kann unmittelbar auf dem Grundkörper des Implantats aufgebracht sein, es können aber auch weitere Schichten zwischen Grundkörper und erfindungsgemäßer Beschichtung vorliegen.

Die Beschichtung des erfindungsgemäßen Implantates umfasst oder besteht aus mindestens einer Schicht, die in einer Lipiddoppelschicht eingebettete Ionenkanäle aufweist und bedeckt bevorzugt die gesamte Oberfläche des Implantats.

Unter einem Ionenkanal wird dabei eine Struktur verstanden, die ein Lumen oder eine Pore bildet, durch welche sich Ionen bewegen oder bewegt werden können. Ist ein Ionenkanal z.B. in einer Lipiddoppelschicht eingebettet, so liegt der Ionenkanal in der Lipiddoppelschicht derart gerichtet vor, dass durch das Lumen oder die Pore des Ionenkanals ein Durchtritt von Ionen von einer Seite der Lipiddoppelschicht auf die andere Seite der Lipiddoppelschicht ermöglicht ist. Dabei weist ein Ionenkanal meist amphiphilen Charakter auf, wobei auf der der Lipiddoppelschicht zugewandten Außenseite nicht-polare Reste überwiegen und auf der dem Lumen zugewandten Seite polare und/oder geladene Reste vorhanden sind. Der Ionentransport durch einen Ionenkanal erfolgt dabei entlang eines bestehenden elektrochemischen Gradienten, dem Konzentrations- und Potentialgefälle. Der Transport von einer Seite der Lipiddoppelschicht auf die andere Seite erfolgt also passiv, ohne dass dazu ein zusätzlicher Energieeintrag notwendig ist.

Ein Ionenkanal besteht meist aus mehreren Molekülen, wobei ein Ionenkanal mehrere verschiedene Untereinheiten aufweisen kann. In einem einfachen exemplarischen Aufbau weist ein Ionenkanal eine Mehrzahl an Molekülen einer ersten Untereinheit auf, die derart "fassartig" angeordnet sind, so dass der Ionenkanal gegenüber der Lipiddoppelschicht, in die er eingebettet ist, abgegrenzt ist und in der Mitte ein Lumen oder eine Pore bildet. Auf der Innenseite des Lumens können sich ein oder mehrere Moleküle einer zweiten Untereinheit befinden, die derart angeordnet und beschaffen sind, so dass ein Transport bevorzugt eines bestimmten Typs von Ionen durch den Ionenkanal ermöglicht ist. Dem Fachmann sind geeignete Ionenkanäle bekannt. Bevorzugt kommen Ionenkanäle zum Einsatz, die aus biokompatiblen Verbindungen und/oder Stoffen bestehen.

Es können beispielsweise Ionenkanäle zum Einsatz kommen, die ganz oder in Teilen Polypeptidstrukturen aufweisen oder daraus bestehen. Unter Polypeptidstrukturen werden Strukturen verstanden, die mindestens 2 proteinogene Aminosäuren umfassen, die miteinander durch eine Peptidbindung verknüpft sind. Peptidstrukturen umfassen Dipeptide, Oligopeptide, Polypeptide und Proteine, sowie Proteinfragmente.

Es können aber auch Ionenkanäle verwendet werden, die peptidfrei sind, also aus organischen Molekülen oder Anordnungen von organischen Molekülen (ggf. enthaltend auch anorganische Moleküle) bestehen, die keine mindestens 2, durch eine Peptidbindung miteinander verbundene proteinogene Aminosäuren aufweisen.

Eine interessante Eigenschaft bestimmter Ionenkanäle ist, dass diese selektiv sein können für den Transport ausgewählter Ionen. In der Beschichtung des erfindungsgemäßen Implantats können ein Teil oder alle Ionenkanäle selektive Ionenkanäle sein. Unter "Selektivität" wird dabei verstanden, dass bevorzugt Ionen mit bestimmten Eigenschaften oder eines bestimmten Typs transportiert werden können, während Ionen, die diese Eigenschaften nicht aufweisen oder von einem anderen Typ sind nicht oder weniger gut transportiert werden. Diese Selektivität kann beispielsweise dadurch erreicht werden, dass das Lumen des Ionenkanals derart beschaffen ist, dass nur Ionen mit einer bestimmten Größe hindurchtreten können, während Ionen, die diese Größe übersteigen am Durchtritt gehindert werden. Die Selektivität eines Ionenkanals kann aber auch durch spezifische Wechselwirkungen zwischen den zu transportierenden Ionen und Bestandteilen des Ionenkanals bewirkt werden.

Beispielsweise können Ionenkanäle eingesetzt werden, die selektiv sind für Kationen, insbesondere selektiv für Ca-, K- und/oder Na-Kationen, besonders bevorzugt für Ca-Kationen mit der Oxidationsstufe +2. Insbesondere können ein Teil oder alle Ionenkanäle eines erfindungsgemäßen Implantats selektiv sein für Kationen, bevorzugt für Ca-Kationen. Besonders bevorzugt weisen Implantate, die ganz oder in Teilen aus einer Magnesiumlegierung bestehen, eine Beschichtung auf, in der ein Teil oder alle Ionenkanäle selektiv sind für Ca-Kationen, bevorzugt Ca²⁺-Ionen.

Ein bevorzugtes Beispiel für einen Ionenkanal, der selektiv ist für den Transport von Kationen insbesondere von Ca-Kationen, ist beschrieben in Das et al. (Proc. Natl. Acad. Sci. USA, 1997, Vol. 94, 9075-9079). Es handelt sich dabei um Ionenkanäle, die das amphiphile Homopolymer Poly(3-hydroxybutyrate) (PHB) umfassen. Bevorzugt umfasst ein PHB-Polymer dabei 115 bis 150 Monomerreste. Besonders bevorzugt werden Ionenkanäle verwendet, die die beiden strukturell unterschiedlichen Polymere PHB und Kalzium-Polyphosphat (Ca(polyP)) umfassen oder daraus bestehen. In den PHB und Ca(polyP) umfassenden Ionenkanälen werden bevorzugt PHB-Homopolymere mit 115 bis 150 Monomerresten verwendet und Ca(polyP) als Ca-Salz von polyP-Anionen mit 50 bis 80 Phosphat-Resten.

Es können aber auch Ionenkanäle eingesetzt werden, die selektiv sind für Anionen, insbesondere selektiv für Cl-, Nitrat und/oder Malat-Anionen, besonders bevorzugt für Cl-Anionen mit der Oxidationsstufe -1. Insbesondere können ein Teil oder alle Ionenkanäle eines erfindungsgemäßen Implantats selektiv sein für Anionen, bevorzugt für Cl-Anionen. Besonders bevorzugt weisen Implantate, die ganz oder in Teilen aus Reineisen oder einer Eisenlegierung bestehen, eine Beschichtung auf, in der ein Teil oder alle Ionenkanäle selektiv sind für Cl-Anionen, bevorzugt C1⁻-Ionen.

Ein bevorzugtes Beispiel für einen Ionenkanal, der selektiv ist für den Transport von Cl-Anionen, ist in Li et al (J. Am. Chem. Soc., 2007, Vol. 129, 7264-7265) beschrieben. Darin wird die Verwendung eines Ionenkanals enthaltend oder bestehend aus einer Mehrzahl von Molekülen der Verbindung 1 offenbart, wobei die Verbindung 1: 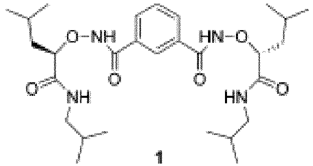 ist.

Im erfindungsgemäßen Implantat kann ein Teil oder können alle Ionenkanäle die Verbindung 1 aufweisen oder aus der Verbindung 1 bzw. einer Mehrzahl davon bestehen:
Ein anderes Beispiel für einen Ionenkanal, der selektiv ist für den Transport von Cl-Anionen, ist beschrieben in Gorteau et al. (J. Am. Chem. Soc., 2006, Vol. 128, 14788-14789). Diese Ionenkanäle zeichnen sich dadurch aus, dass diese stabförmiges oligo-(p-phenylen)-*N,N*-naphthalendiimid (O-NDI) aufweisen oder aus einer Mehrzahl stabförmiger O-NDI-Moleküle bestehen, wobei der Begriff "oligo" für eine Anzahl von Monomeren von 2 bis 20 steht. Dabei sind von dem Begriff "O-NDI" ausdrücklich auch solche stabförmigen oligo-(p-phenylen)-*N,N*-naphthalendiimide umfasst, bei denen eines oder beide endständigen Amine substituiert oder modifiziert sind. So kann beispielsweise ein Wasserstoff eines oder beider endständigen Amine eines O-NDI-Moleküls durch eine Schutzgruppe, beispielsweise durch eine *tert*-Butyloxycarbonyl-Gruppe (BOC) substituiert sein. Es können aber auch eine oder beide endständigen Amine als Ammonium, bevorzugt als Ammoniumsalz, insbesondere als Ammoniumsalz mit Trifluoressigsäure (TFA) vorliegen. Im erfindungsgemäßen Implantat kann ein Teil oder können alle Ionenkanäle ein oligo-(p-phenylen)-*N,N*-naphthalendiimid (O-NDI)-Molekül aufweisen oder aus einem oder einer Mehrzahl von O-NDI-Molekülen bestehen.

Die Beschichtung eines erfindungsgemäßen Implantats kann ausschließlich Ionenkanäle eines einzigen Typs aufweisen oder eine Mischung verschiedener Ionenkanäle, bevorzugt mit unterschiedlicher Selektivität aufweisen.

Im erfindungsgemäßen Implantat liegen die Ionenkanäle in eine Lipiddoppelschicht eingebettet vor. Eine Lipiddoppelschicht ist eine Struktur, die von einer Vielzahl von amphiphilen Lipiden in Anwesenheit eines polaren Lösungsmittels ausgebildet wird, wobei sich eine Membran ausbildet, bei der der hydrophobe Teil der amphiphilen Lipide den Kern der Membran bildet und der hydrophile Teil die die Membran begrenzende Außenseiten darstellt. Eine wichtige Eigenschaft von Lipiddoppelschichten besteht darin, dass diese nahezu undurchlässig sind für polare Moleküle oder Makromoleküle, gleichzeitig aber sehr flexibel und mechanisch schwer zu zerstören sind. Bevorzugt weist die Lipiddoppelschicht amphiphile Lipide auf oder besteht daraus. Besonders bevorzugt sind dies biokompatible amphiphile Lipide. Ganz besonders bevorzugt enthält oder besteht die Lipiddoppelschicht aus Phospholipiden, Phosphoglyceriden, Sphingolipiden, Phosphatidylethanolaminen, Phosphatidylserinen, Phosphatidylcholinen, Sphingomyeline und/oder Plasmalogenen, sowie Mischungen davon.

Aufgrund ihres amphiphilen Charakters können sich die Ionenkanäle ggf. eigenständig in der Lipiddoppelschicht organisieren und ausrichten, so dass die Ionenkanäle in funktionsfähiger Orientierung vorliegen und einen Transport von Ionen zwischen den beiden Seiten der Lipiddoppelschicht erlauben.

Verfahren zum Anbringen einer Beschichtung umfassend eine Schicht, die in eine Lipiddoppelschicht eingebettete Ionenkanäle aufweist sind dem Fachmann grundsätzlich bekannt. Bevorzugte Verfahren umfassen Tauchen, Beschichten in einem Langmuir-Blodgett-Verfahren, Sprühen, "self assembly"-Verfahren, Rotationsbeschichtung und/oder "drop casting"-Verfahren.

Die erfindungsgemäße Beschichtung kann weitere Stoffe, insbesondere pharmazeutische Wirkstoffe, Röntgenmarker oder Magnetresonanzmarker enthalten.

Nachfolgend wird die Erfindung anhand von Ausführungsbeispielen näher erläutert.

### Beispiel 1:

Es wurde eine Lösung des Komplexes aus Poly(3-hydroxybutyrat) (PHB) und anorganischem Polyphosphat (polyP) in Chloroform hergestellt, in dem eine Lösung von PHB (1µg/ml) in Chloroform mit Ca(polyP) versetzt wird, welches zuvor aus Natriumphosphat und CaCl hergestellt wurde.

Der PHB-polyP-Polymerlösung wurde eine Lösung von Dimyristoyl-phosphatidylcholin (DMPC) in Chloroform mit DMPC in einer Konzentration von 0.9 mg/ml zugesetzt. In einer Langmuir-Blodget Vorrichtung werden Tropfen (20 µl) der hergestellten Lösung zufällig über eine wässrige Subphase verteilt. Der entstandene flottierende Film wurde durch zwei mobile Platten mit einer Rate von 5 mN m⁻¹ min⁻¹ linear komprimiert. Der erhaltene Film wurde durch Abstreifen auf einen Stent übertragen.

### Beispiel 2:

### (Vergleichsbeispiel, nicht unter die Erfindung fallend)

Ein Stent wurde durch 3-minütiges Tauchen in der PHB-polyP-Polymerlösung aus Beispiel 1 mit einer Ionenkanäle aufweisenden Beschichtung versehen. Der derart beschichtete Stent wurde anschließend luftgetrocknet.

## Patentansprüche

1. Implantat, welches ganz oder in Teilen aus einem biokorrodierbaren metallischen Werkstoff besteht und eine Beschichtung aufweist, **dadurch gekennzeichnet, dass** die Beschichtung mindestens eine Schicht enthält, in die Ionenkanäle eingebettet sind, wobei die Ionenkanäle in eine Lipiddoppelschicht eingebettet vorliegen.

2. Implantat nach Anspruch 1, **dadurch gekennzeichnet, dass** das Implantat ein Stent ist.

3. Implantat nach einem der vorhergehenden Ansprüche, **dadurch gekennzeichnet, dass** der biokorrodierbare metallische Werkstoff eine Magnesiumlegierung ist.

4. Implantat nach einem der Ansprüche 1 oder 2, **dadurch gekennzeichnet, dass** der biokorrodierbare metallische Werkstoff Eisen oder eine Eisenlegierung ist.

5. Implantat nach einem der vorhergehenden Ansprüche, **dadurch gekennzeichnet, dass** die mindestens eine Schicht ausschließlich Ionenkanäle eines einzigen Typs aufweist.

6. Implantat nach einem der vorhergehenden Ansprüche, **dadurch gekennzeichnet, dass** ein Teil oder alle Ionenkanäle Polypeptidstrukturen aufweisen.

7. Implantat nach einem der Ansprüche 1 bis 5, **dadurch gekennzeichnet, dass** ein Teil oder alle Ionenkanäle peptidfrei sind.

8. Implantat nach einem der vorhergehenden Ansprüche, **dadurch gekennzeichnet, dass** ein Teil oder alle Ionenkanäle selektiv sind.

9. Implantat nach einem der vorhergehenden Ansprüche, **dadurch gekennzeichnet, dass** ein Teil oder alle Ionenkanäle selektiv sind für Kationen, bevorzugt Ca-Kationen.

10. Implantat nach einem der Ansprüche 1 bis 8, **dadurch gekennzeichnet, dass** ein Teil oder alle Ionenkanäle selektiv sind für Anionen, bevorzugt Cl-Anionen.

11. Implantat nach Anspruch 9, **dadurch gekennzeichnet, dass** ein Teil oder alle Ionenkanäle poly(3-Hydroxybutyrat) (PHB) aufweisen oder daraus bestehen, bevorzugt PHB und Kalzium-Polyphosphat (Ca(polyP)), besonders bevorzugt PHB-Polymere mit 115- 150 Monomerresten und Ca(polyP) mit 50 - 80 Phosphat-Resten.

12. Implantat nach Anspruch 10, **dadurch gekennzeichnet, dass** ein Teil oder alle Ionenkanäle eine Mehrzahl der Verbindung 1 aufweisen oder daraus bestehen, wobei die Verbindung 1: 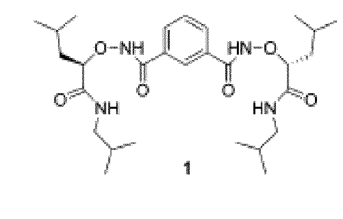 ist.

13. Implantat nach Anspruch 10, **dadurch gekennzeichnet, dass** ein Teil oder alle Ionenkanäle ein oligo-(p-phenylen)-NN-naphthalendiimid (O-NDI)-Molekül aufweisen oder aus einem oder einer Mehrzahl von O-NDI-Molekülen bestehen.

14. Implantat nach einem der vorhergehenden Ansprüche, **dadurch gekennzeichnet, dass** die Lipiddoppelschicht amphiphile Lipide aufweist oder daraus besteht, bevorzugt biokompatible amphiphile Lipide aufweist oder daraus besteht, besonders bevorzugt Phospholipide, Phosphoglyceride, Sphingolipide, Phosphatidylethanolamine, Phosphatidylserine, Phosphatidylcholine, Sphingomyeline und/oder Plasmalogene, sowie Mischungen davon aufweist oder daraus besteht.

## Claims

1. An implant, which is made entirely or in parts from a biocorrodible metallic material and comprises a coating, **characterized in that** the coating contains at least one layer in which ion channels are embedded, wherein the ion channels are embedded in a lipid bilayer.

2. The implant according to claim 1, **characterized in that** the implant is a stent.

3. The implant according to any one of the preceding claims, **characterized in that** the biocorrodible metallic material is a magnesium alloy.

4. The implant according to any one of claims 1 or 2, **characterized in that** the biocorrodible metallic material is iron or an iron alloy.

5. The implant according to any one of the preceding claims, **characterized in that** the at least one layer exclusively comprises ion channels of a single type.

6. The implant according to any one of the preceding claims, **characterized in that** a portion or all of the ion channels have polypeptide structures.

7. The implant according to any one of claims 1 to 5, **characterized in that** a portion or all of the ion channels are peptide-free.

8. The implant according to any one of the preceding claims, **characterized in that** a portion or all of the ion channels are selective.

9. The implant according to any one of the preceding claims, **characterized in that** a portion or all of the ion channels are selective for cations, preferably Ca cations.

10. The implant according to any one of claims 1 to 8, **characterized in that** a portion or all of the ion channels are selective for anions, preferably Cl anions.

11. The implant according to claim 9, **characterized in that** a portion or all of the ion channels comprise poly(3-hydroxybutyrate) (PHB) or consist thereof, preferably PHB and calcium polyphosphate (Ca(polyP)), particularly preferably PHB polymers having 115 - 150 monomer groups and Ca(polyP) having 50 - 80 phosphate groups.

12. The implant according to claim 10, **characterized in that** a portion or all of the ion channels comprise a plurality of compound 1 or consist thereof, wherein compound 1 is:

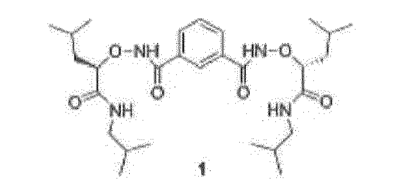

13. The implant according to claim 10, **characterized in that** a portion or all of the ion channels comprise an oligo-(p-phenylene)-*N,N*-naphthalenediimide (O-NDI) molecule or consist of one or a plurality of O-NDI molecules.

14. The implant according to any one of the preceding claims, **characterized in that** the lipid bilayer comprises or consist of amphiphilic lipids, preferably comprises or consist of biocompatible amphiphilic lipids, particularly preferably phospholipids, phosphoglycerides, sphingolipids, phosphatidylethanolamines, phosphatidylserines, phosphatidylcholines, sphingomyelins, and/or plasmalogens, and comprises or consist of mixtures thereof.

## Revendications

1. Implant, lequel est totalement, ou en partie, constitué d'un matériau métallique bio-corrodable et présente un revêtement, **caractérisé en ce que** le revêtement contient au moins une couche dans laquelle les canaux ioniques sont intégrées, où les canaux ioniques sont présents intégrés dans une double couche de lipides.

2. Implant selon la revendication 1, **caractérisé en ce que** l'implant est une prothèse endovasculaire.

3. Implant selon l'une des revendications précédentes, **caractérisé en ce que** le matériau métallique bio-corrodable est un alliage de magnésium.

4. Implant selon l'une des revendications 1 ou 2, **caractérisé en ce que** le matériau métallique bio-corrodable est du fer ou un alliage de fer.

5. Implant selon l'une des revendications précédentes, **caractérisé en ce que** la au moins une couche présente exclusivement des canaux ioniques d'un seul type.

6. Implant selon l'une des revendications précédentes, **caractérisé en ce qu'**une partie, ou la totalité, des canaux ioniques présente des structures polypeptidiques.

7. Implant selon l'une des revendications 1 à 5, **caractérisé en ce qu'**une partie, ou la totalité, des canaux ioniques est exempte de peptides.

8. Implant selon l'une des revendications précédentes, **caractérisé en ce qu'**une partie, ou la totalité, des canaux ioniques est sélective.

9. Implant selon l'une des revendications précédentes, **caractérisé en ce qu'**une partie, ou la totalité, des canaux ioniques est sélective pour des cations, de préférence des cations Ca.

10. Implant selon l'une des revendications 1 à 8, **caractérisé en ce qu'**une partie, ou la totalité, des canaux ioniques est sélective pour des anions, de préférence, des anions Cl.

11. Implant selon la revendication 9, **caractérisé en ce qu'**une partie, ou la totalité, des canaux ioniques présente du poly (3-hydroxybutyrate) (PHB) ou en est constituée, de préférence du PHB et du polyphosphate de calcium (Ca(polyP)), de manière particulièrement préférée des polymères PHB avec 115 à 150 unités monomères et du Ca(polyP) avec 50 à 80 unités phosphates.

12. Implant selon la revendication 10, **caractérisé en ce qu'**une partie, ou la totalité, des canaux ioniques présente une multiplicité de composés 1 ou en est constituée, où le composé 1 est :

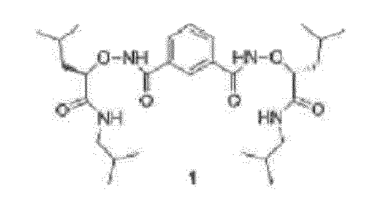

13. Implant selon la revendication 10, **caractérisé en ce qu'**une partie, ou la totalité, des canaux ionique présente une molécule d'oligo-(p-phénylène)-N,N-naphthalènediimide (O-NDI), ou est constituée par une ou une multiplicité de molécules O-NDI.

14. Implant selon l'une des revendications précédentes, **caractérisé en ce que** la double couche de lipides présente des lipides amphiphiles ou en est constituée, présente de préférence des lipides amphiphiles biocompatibles ou en est constituée, de manière particulièrement préférée, présente des phospholipides, des phosphoglycérides, des sphingolipides, de la phosphatidyléthanolamine, de la phosphatidylsérine, de la phosphatidylcholine, de la sphingomyéline et/ou du plasmalogène, ainsi que des mélanges de ceux-ci, ou en est constituée.
